(19) Europäisches Patentamt / European Patent Office / Office européen des brevets

(11) **EP 2 950 769 B1**

(12) **EUROPEAN PATENT SPECIFICATION**

(45) Date of publication and mention
of the grant of the patent:
**28.11.2018 Bulletin 2018/48**

(51) Int Cl.:
**G16H 20/30** *(2018.01)*     *A61H 9/00* *(2006.01)*

(21) Application number: **14746414.3**

(22) Date of filing: **30.01.2014**

(86) International application number:
**PCT/SG2014/000042**

(87) International publication number:
**WO 2014/120094 (07.08.2014 Gazette 2014/32)**

(54) **A GARMENT FOR TREATING SENSORY DISORDER**

KLEIDUNGSSTÜCK ZUR BEHANDLUNG VON SINNESSTÖRUNG

VÊTEMENT POUR TRAITER UN TROUBLE SENSORIEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2013  SG 201300831**
**28.02.2013  PCT/SG2013/000081**

(43) Date of publication of application:
**09.12.2015  Bulletin 2015/50**

(73) Proprietors:
• **Lai, Sep Riang**
**Singapore 680437 (SG)**
• **Lin, Wei Liang**
**Singapore 510503 (SG)**
• **Teh, Keng Soon**
**Singapore 140157 (SG)**

(72) Inventors:
• **Lai, Sep Riang**
**Singapore 680437 (SG)**

• **Lin, Wei Liang**
**Singapore 510503 (SG)**
• **Teh, Keng Soon**
**Singapore 140157 (SG)**

(74) Representative: **Talbot-Ponsonby, Daniel Frederick**
**Marks & Clerk LLP**
**Fletcher House**
**Heatley Road**
**The Oxford Science Park**
**Oxford OX4 4GE (GB)**

(56) References cited:
JP-A- 2003 275 263     JP-A- 2012 526 605
US-A- 5 490 820     US-A1- 2006 122 544
US-A1- 2008 222 771     US-A1- 2008 222 771
US-A1- 2010 292 619     US-B2- 6 752 771

**Description**

**Field**

The present invention relates to a garment for treating a sensory disorder such as anxiety attacks for people with sensory disorder, such as people with autism spectrum disorder or attentional difficulties.

**Background**

It is known to provide a deep pressure touch simulation garment, such as that disclosed in European Patent publication number 2355767. This includes an internal harness that supports a number of controllable air bladders. An external garment attaches over the harness. However it is not possible to independently control the bladders or to control it remotely using a smart phone device, nor is it highly wearable. Other prior art examples are given in US20040054306, WO20080314, US5437610, WO2011084709, US20120284898, WO2009114822, KR20120102434, KR20120078760, US2002089304, US2006242746, KR20120034095, US6086551, US2003074711, US2010199405, US2003230474 and CN201541756. US2008222771 and US2008222769 disclose a pressure-providing garment; US2010292619 discloses a performance-enhancing method; US20060122544 discloses a therapeutic garment; US6752771 describes an inflatable vest; US5490820 discloses a cardiac assist device.

**Summary**

The scope of the invention is defined by the appended claims. In general terms in an embodiment the present invention proposes a highly wearable garment with a plurality of integrated air bladders which can be independently pressure controlled. This may have the advantage of maximising therapeutic efficacy of a pressure garment for people with autism spectrum disorder or attentional difficulties, such as autistic children, during an anxiety attack, when they are hyperactive, experiencing attentional difficulties, or when they are hypoactive. The variable pressure on different body portions may be prescribed after diagnosis by a clinician or healthcare professional, the symptoms remotely monitored and/or the prescribed treatment revised in view of monitoring.

In an embodiment the invention proposes a smart phone app that connects to a garment for treating sensory disorder, and allows the user to remotely control the pressure applied and/or select automatic therapy of static or varying pressure based on the user's activity, motion or physiological parameters.

An example, not being part of the invention, proposes an Autism Community Portal which allows teachers, therapists and/or parents to access historical usage data and/or modify the treatment schedule for one or more users. This may have the advantage that information about different children with autism, their profiles and treatments received can be shared which may promote a community driven approach towards the best practice among individual families and caregivers.

In an embodiment the invention proposes a method of determining an activity index. The index may be based on the users body temperature, heart rate and/or sound level adjacent the user. This may have the advantage that the user's arousal level and indication about condition of the children can be tracked and/or automatically used to determine whether or what kind of treatment to provide.

An example, not being part of the invention, proposes a method of recording a child's behavioural progress and a contextual situation on an app after each pressure therapy session. This information may then be uploaded onto an autism community portal, allowing caregivers to monitor the progress of the child.

In an embodiment, the invention proposes a highly wearable garment with embedded electronics. The garment may be light weight and comfortable for the user despite the embedded electronics and pneumatics system.

In a first aspect of the invention there is provided a garment as claimed in claim 1. Embodiments may be implemented according to any of claims 2 to 12.

In a second aspect of the invention there is provided a remote application or web interface for interaction with a garment for treating a sensory disorder according to claim 13.

**Brief Description of Drawings**

One or more example embodiments of the invention will now be described, with reference to the following figures, in which:

Figure 1 is a block diagram showing a system according to an example embodiment;
Figure 2a is a flow diagram showing the interaction between the User, App, Cloud and Jacket;
Figure 2b is a flow diagram showing the interaction between the Cloud and the Therapist (Caregiver);

Figure 3 is Circuit Diagram of the Hardware Master;
Figure 4 is a circuit Diagram of the Hardware Slave;
Figure 5 is a Flowchart of the Master Unit Firmware;
Figure 6 is a Flowchart of the Air Bladder Update Routine;
Figure 7 is a Use Case Diagram of the Software Use;
Figure 8 is a photo of the bladders providing Upper body inwards pressure;
Figure 9 is a photo of the bladders providing inwards pressure and constrictive effects (Upper and lower body);
Figure 10 is a photo of the Adjustment strap;
Figure 11 is a photo of the bladders avoiding pressure on diaphragm area;
Figure 12 is a schematic diagram of the Anti vibration measures;
Figure 13 is a photo of the Removable belt design for easy washing;
Figure 14 is a schematic diagram of the Power button holder;
Figure 15 is a schematic diagram of the Power button pocket;
Figure 16 is a photo of the Configurations and Positions of electronic components (3 units);
Figure 17 is a photo of the Smaller airbag channels under main controller unit;
Figure 18 is a flow chart of the app UI login;
Figure 19 is a screen shot of the app UI main page;
Figure 20 is a screen shot of the app UI manual therapy page;
Figure 21 is a screen shot of the app UI auto therapy page;
Figure 22 is a screen shot of the app UI play profile page;
Figure 23 is a screen shot of the app UI edit profile page;
Figure 24 is a screen shot of the app UI community page;
Figure 25 is a screen shot of the app UI profile edit page;
Figure 26 is a screen shot of the app UI upload photo/video page;
Figure 27 is a screen shot of the app UI upload pressure profile page;
Figure 28 is a screen shot of the app UI magnified pressure profile view page;
Figure 29 is a screen shot of the app UI comments view page;
Figure 30 is a screen shot of the app UI other users view page;
Figure 31 is a schematic diagram showing the cloud architecture data flow diagram;
Figure 32 a schematic diagram showing the data collected from the user;
Figure 33 is a flow diagram of the determination of the activity index;
Figure 34 is a screen shot if the Web UI summary page;
Figure 35 is a screen shot if the Web UI custom therapy page;
Figure 36 is a screen shot if the Web UI preset therapy page;
Figure 37 is a flow diagram of the automated addressing method for slave;
Figure 38 is a photo of pleats in the should bladder; and
Figure 39 is a photo of the pleats in the chest bladder.

**Detailed Description**

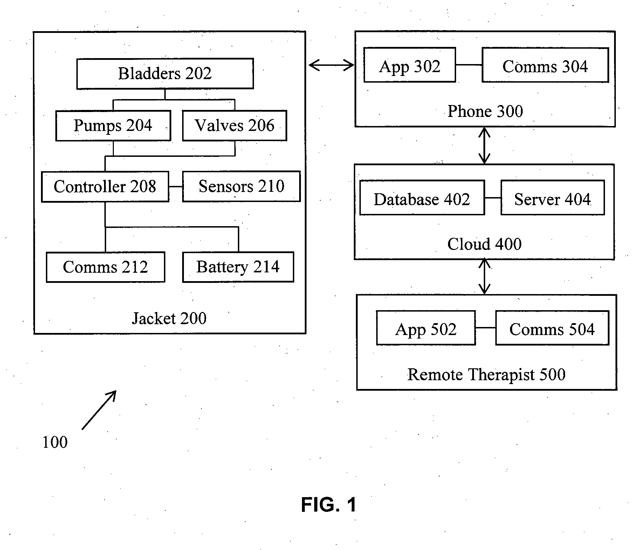

A system 100 according to an example, not being part of the invention, will now be described with reference to Figure 1. The system 100 includes a garment 200, a user control app (loaded on a mobile phone or mobile device 300), a remote cloud 400, and a remote app or web interface 500 used by the therapist. The user can control the garment 200 using the app 300. The user's settings and the user's physiological parameters are monitored via the user phone 300 and stored in the cloud 400. In turn the therapist 500 can monitor the physiological parameters or change settings.

The garment 200 may be a jacket which incorporates multiple air bladders 202, batteries 214, a controller 208 including sensors 210 and valves 206, a pump 204 energised by the batteries 214 according to the controller 208. One sensor may be provided for each air bladder to detect the pressure in each air bladder. A communications module 212 connects the controller 208 to the phone 300. As mentioned above each bladder may be independently pressure controlled. The garment may also be a vest, shirt, sweater, wrap around, shoulder strap, armband, backpack, long sleeve shirt / jacket, dress, blouse, hooded or unhooded, that covers any upper body part and/or arm areas.

The phone 300 includes app 302 which may downloadable from an app store and installable into a mobile device operating system such as Andriod, iOS, WindowPhone BlackBerry etc. The app 302 includes a user interface and protocols to communicate via the phone/tablet etc 300 native communications processors 304, eg: Bluetooth. The phone 300 includes a screen that receives input from the user and displays a status of the garment 200. The screen may be a touch screen.

The app 302 stores data from the garment 200 and the app 302 periodically transfers this data to the cloud 400. The cloud 400 includes a webserver 404 which communicates with each app 302 via a secure https channel. A database 402 stores the data from all of the users, and includes security protocols to ensure data privacy.

The remote user 500 may include a therapist, medical professional, parents or governmental concerns. A remote user app 502 includes the functionality to monitor user in real-time or review historical trends in the check on the physiological parameters. The therapist can select from a range of pre-set therapies, or may customise a specific therapy for each user. For example the app 302 may be programed to automatically respond to a particular physiological parameter breaching a threshold by providing different pressures to specific bladders. Later after reviewing the user's response to the therapy, the therapist may revise the settings. The remote app may be via a web page or via an app loaded on a mobile phone.

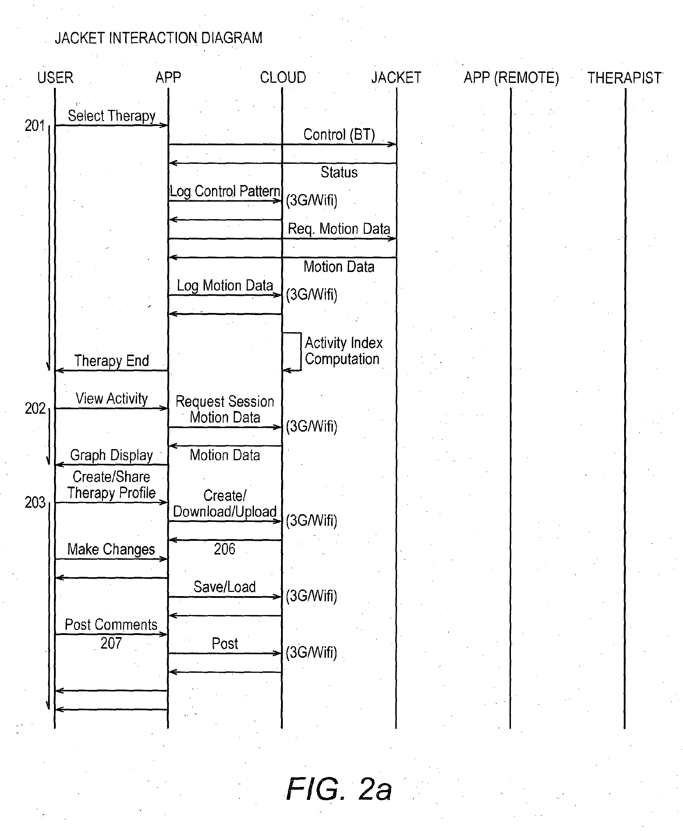

Figure 2a shows the interaction between the user app 300 and the cloud 400.

1. A user can use the app to start a therapy session 201. The Jacket can be manually controlled via app to give the right pressure profile to the body. User's control pattern on the app and motion feedback from the Jacket will be logged on the Cloud for analytics. The data will be further compute into an Activity Index that may be related to user's stress or arousal level.

2. The user can also choose to view the Activity Index 202 computed in graphical format.

3. With the feedback that reflect effectiveness of the therapy profile applied 203, the user can create and share the profile to other users through saving and uploading it 206 to the social networking platform integrated to the Cloud. All users that the profile is shared to can make comments 207 with regard to the profile.

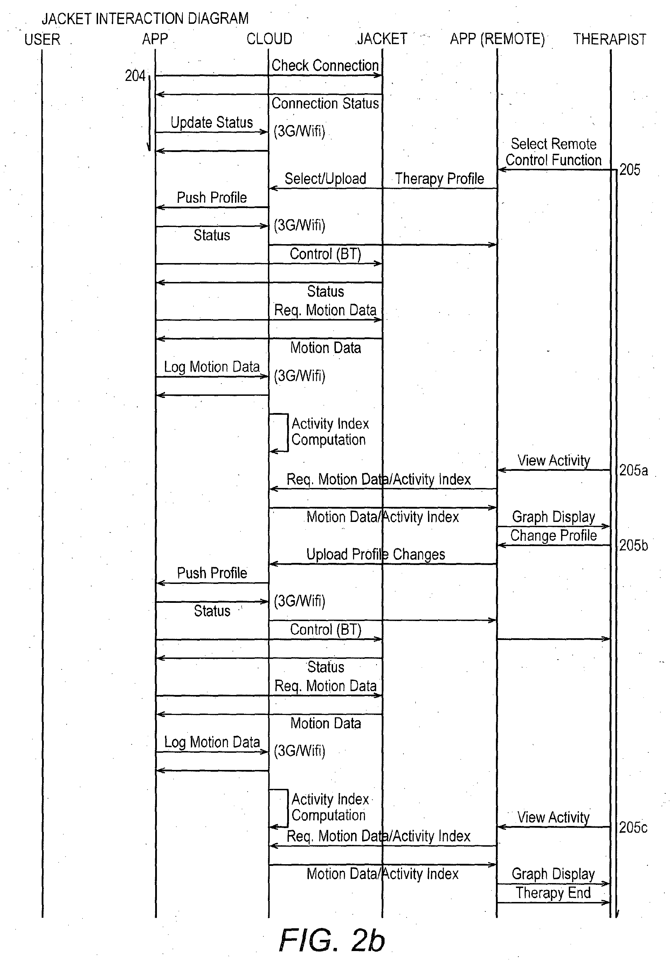

Figure 2b shows the interaction between the therapist 500 and the cloud 400:

4. The app will periodically update the Cloud with the connected Jacket status 204 so that remote user will be able to know whether the Jacket is ready to be controlled remotely.

5. Therapist or parents can remote control a Jacket anywhere they want by using the app 205.

    a. They can view the real time Activity Index 205a of the Jacket wearer via the app.

    b. They can make changes to the therapy profile 205b that will be applied to the wearer based on the feedback they have get.

    c. After making profile changes, they can view the response again 205c and further adjust the profile until satisfactory result is observed.

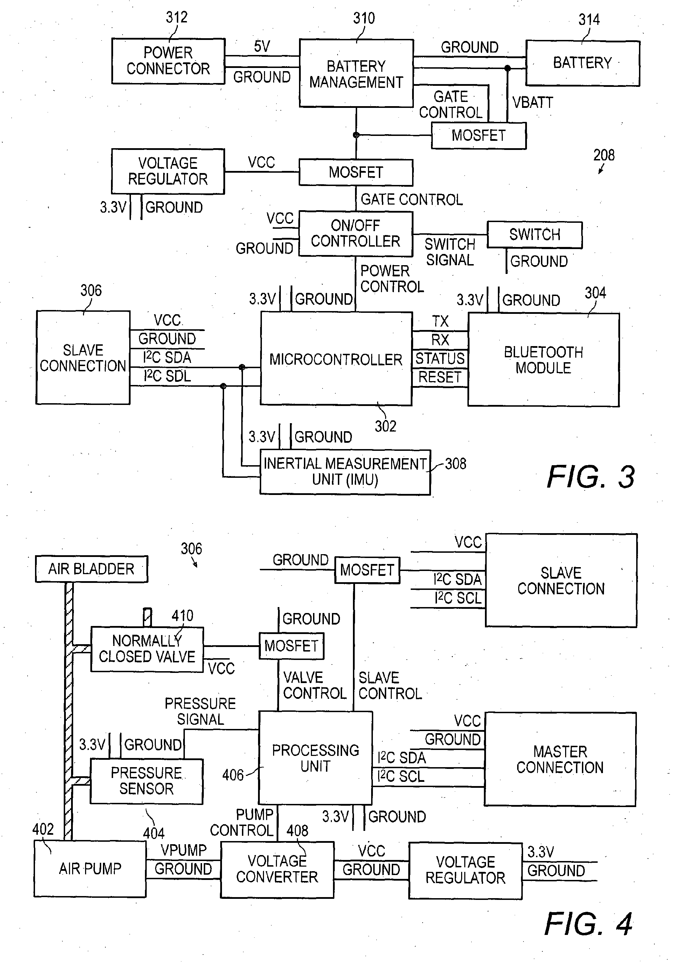

The controller 208 may be implemented as shown in Figures 3 and 4. The master unit contains the main controller 302 that controls the pressure to be applied to the Jacket wearer. It receives commands from the Jacket app via Bluetooth 304 and instructs the slave circuit 306 to inflate/deflate the air bladders to the right pressure level. It senses user's motion through the inertial measurement unit 308. A battery and battery management circuit are integrated into the system to

- Battery Management 310 - Usage while charging is allowed. Low heat generation to ambient environment.
- Power Connector 312 - May utilise USB connector for charging purposes for size optimization and ease of use.
- Battery 314 - Flat rechargeable Lithium Battery is used to optimize power over weight ratio of the system.
- Bluetooth - Can be upgraded to Bluetooth Low Energy to allow low wireless communication power consumption.
- Inertial Measurement Unit - Consists of accelerometer and/or gyroscope and/or magnetometer.
- Master unit may combine with slave units to form an integrated system. Integration can be done via eliminating processing unit at slave units and its processing control will be taken over by microcontroller at master unit.
- Slave Connection - Inter circuit connection to the slave unit is digital to reduce signal deterioration due to noise.

As shown in Figure 4 each slave unit 306 consists of one actuator (pump) 402, one sensor (pressure transducer) 404 and control 406 to form a closed loop control system.

- General - Plurality of slave modules allowed in the following daisy-chain connection

| Master | -------- | Slave 1 | -------- | Slave 2 | -------- | Slave 3 |

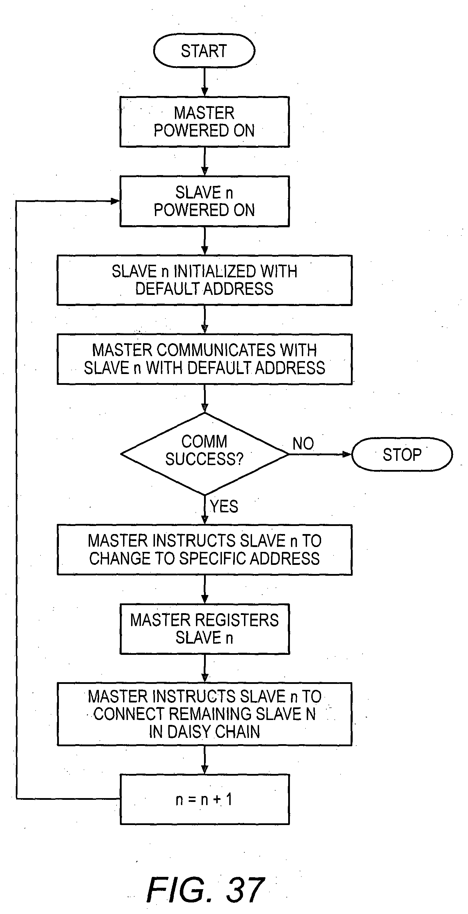

- Addresses to identify slave unit do not need to be specified by hardware nor by firmware. A method for assigning unique address to slave and registering the address with Master is shown in Figure 37. This allows end users to freely customize number of slave units. The user can then decide how many bladders to connect to each slave units, and connect them appropriately using the tubing. For example each slave pump may have multiple outlet ports, or Y connectors may be used.

- The system will be able to detect number of Slave units attached to the Master unit. This information will be sent to the mobile device paired with the system. Mobile device displays different controlling user interface based on this information. Users might have to configure the app user interface manually so that all Slave units are matched to air bladders that are in various location on body.
- Modularize system allows weight distributed more evenly on wearer's body.
- Slave/Master Connection - Inter circuit connection to the other unit is digital to reduce signal deterioration due to noise.
- Voltage Converter 408 - Dedicated voltage converter for each slave unit allows multi-slave expansions without having the need to change the design of the master unit.
- Normally Closed Valve 410 - The valve is chosen to be normally closed to reduce power consumption while holding the pressure in the air bladder. The air pressure of the air bladder should remain constant most of time during the therapy session. An exhausting pump may be required if the deflation duration is too long. The system will monitor for any leaks and inform the therapist and/or user in case of any malfunctions requiring intervention. Similarly the valves may selectively connected to a single bladder or multiple bladders, depending on the users desired operating configuration.
- Air pump 402 - Air flow silencer will be required to reduce operational noise.
- Air pump 402 - Rolling pump with three diaphragm chambers is chosen to optimize the size, noise and vibration of the system.
- Pressure sensor 404 - Pressure sensor is chosen to have sensing range between 0 kPa and 40 kPa to maximize sensing precision.
- Processing Unit 406 - Processing unit may just consist of analogue to digital converter and logic input of output to simplify production process.
- Inertial Measurement Unit 308 - Modularized system allows IMU to be placed away from Air pump 402, which is a vibrational source, to minimize noise in motion readings.

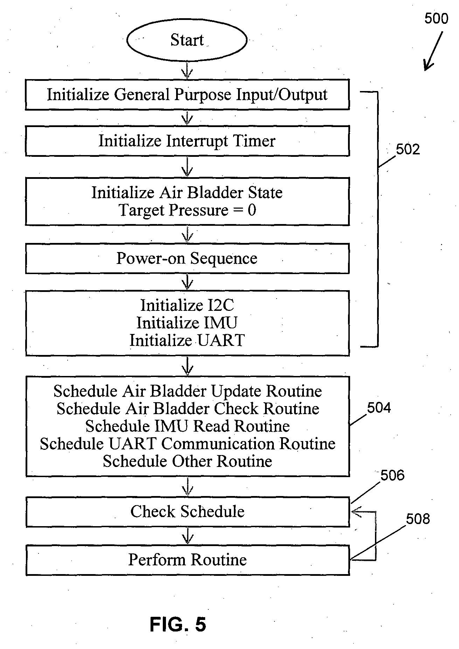

The controller 208 has main operating algorithm 500 according to the firmware shown in Figure 5. All of the variables and routines are initialised 502 upon turnon. The various pressure update routines and check sequences are then scheduled 504. The schedule is then checked 506 or updates by the user/therapist. Any routines are then performed 508 according to the schedule.

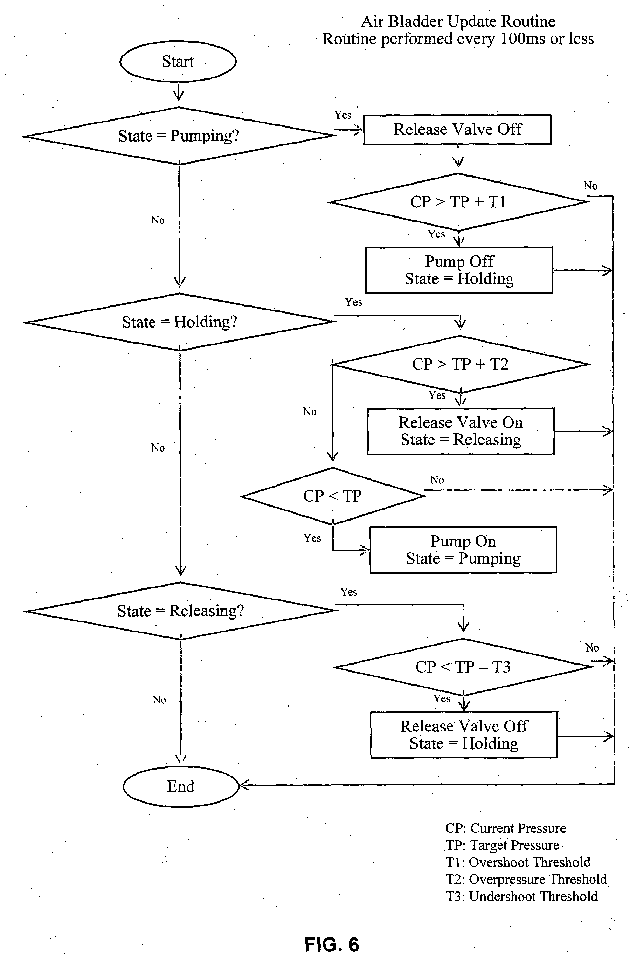

As shown in Figure 6 the Air Bladder Update Routine 504 manages the pressure of each bladder using the pressure sensors according to the set point for each bladder. There is also an Air Bladder Check routine which reads the pressure sensor value for each bladder and stores the values. The IMU Read routine reads the inertial measurement unit sensor values and stores the values. The UART Communication routine reads the incoming data from the Bluetooth module and store the data, and writes data to the Bluetooth module. Each of the tasks may have a different period to ensure even controller load distribution and task can be performed in a timely manner.

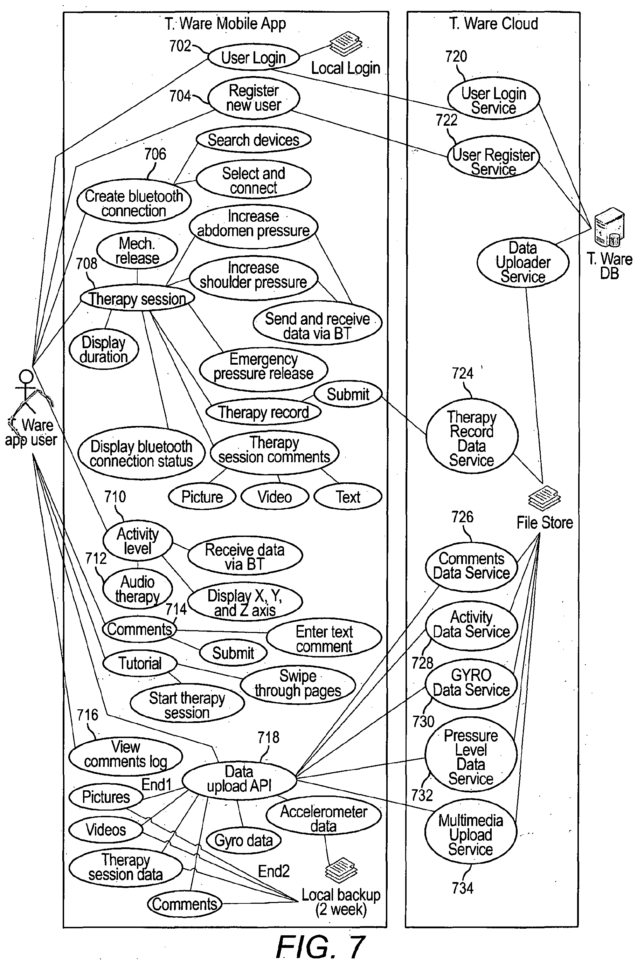

Figure 7 illustrates how users can interact with the system. Users can (from top to bottom):

1) Login to system (access to Cloud) 702
2) Register new user 704
3) Establish Bluetooth connection between Mobile phone and Jacket 706
4) Start therapy session (control pressure level & location) 708
5) View activity level (through motion sensor data from the Jacket) 710. Auto therapy 712 will trigger the appropriate pressure therapy based on activity level detected. The system may also be configured to be in manual therapy mode where an alert notification will be sent to the therapist to give them the option to provide pressure therapy
6) Enter comments 714
7) View comments 716
8) Upload data on the phone to the cloud server to be stored online 718

There are services designed on the cloud server to communicate with the mobile phone app and the database.

1) User Login Service 720: interface between user login data and server authentication
2) User Register Service 722: interface between new user registration and server database
3) Therapy Record Data Service 724: enables system to save user feedback at the end-of the therapy session through a structured form.
4) Comments Data Service 726, Activity Data Service 728, Gyro Data Service 730, Pressure Level Data Service 732, Multimedia Upload Service 734: enables user to save comments, activity, gyro, pressure level, and multimedia (pictures, videos) on the database.

The bladders 202 include a number of separately controlled bladders. Each bladder is made by having two layers of thermoplastic polyurethane (TPU) like material placed together and heat sealing them with a metal mould placed over the TPU layers. The mould is shaped accordingly to a form of desired air channel configuration within each bladder. In this way, two air channel configurations are fabricated, one for the upper body and one of the lower body of the user. In use, the air channel configuration for the upper body is for the upper back, shoulders and chest area of the user and the air channel configuration for the lower body is for the lower back and abdomen area of the user. The air channels are sewn onto an inner fabric lining of the jacket. When seals are made within an air bladder but not sealing off any part of the bladder completely, air channels are formed. The purpose of the air channels is to reduce the bulging effect of the air bladder for aesthetic reasons and also to produce a constricting effect when the air channels are made in a certain configuration.

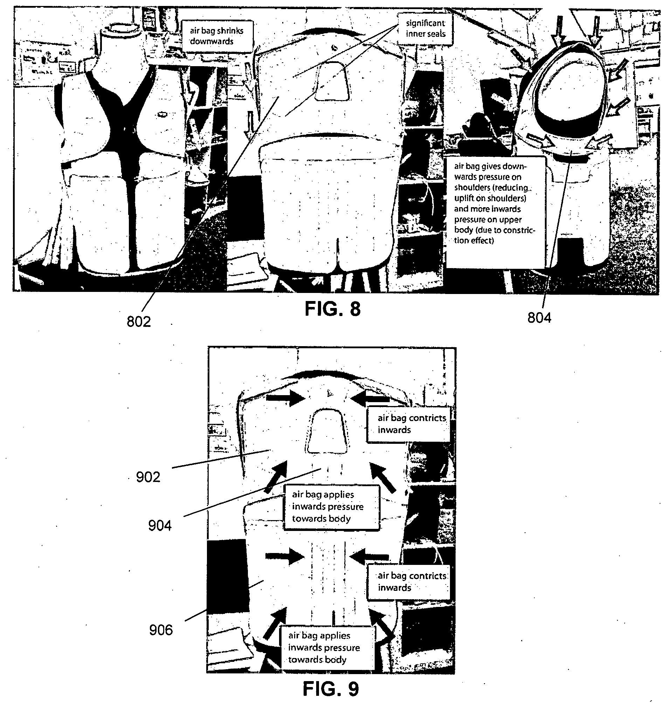

Figure 8 shows the bladders demonstrating an inwards pulling effect (in the direction perpendicular to the long strip seals). The bladders have significant inner seals 802 formed by bonding of the TPU layers through heat sealing, so that the shoulder area can be constricted inwards, giving pressure around and on the shoulders. A downwards pressure on the shoulders is further enhanced due to the sufficient anchorage provided by traction from the inflated bladder 804 under the arm pit. This constriction inwards also reduces the uplifting of the airbags on shoulder region, and more inward pressure can be applied on the upper chest and upper back areas.

Figure 9 shows a mixture of small 902 and large channels 904 within the air bladders for an all round tightening pressure and inwards pushing effects at pressure sensitive areas. This results in greater all round pressure and inwards pressure at pressure sensitive areas 906.

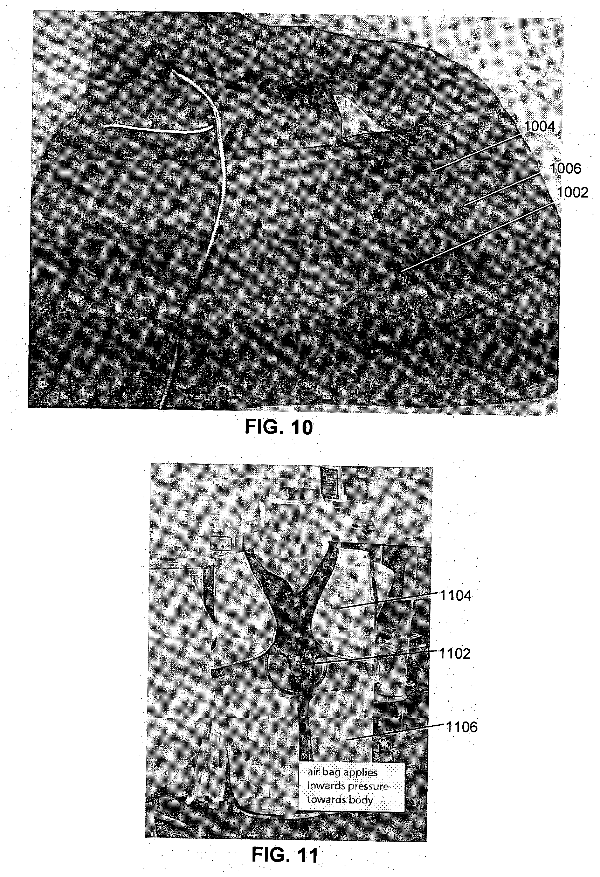

Figure 10 shows the use of a double D ring 1002 size adjustment strap 1004 to better fit the jacket to the user. The adjustment straps 1004 are located on the side in contact with the body and not within the jacket layers, allowing easy pulling of the straps to adjust the jacket to fit user. By pulling the straps, the inner lining 1006 of the jacket will be pulled in to fit the user. This helps to ensure that the user can feel the pressure from the airbags more effectively. This adjustment is easy to make and automatically locks compared to a hook and fastener adjustment.

Figure 11 shows the bladders are designed to avoid the diaphragm area 1102 and yet applying sufficient pressure on upper chest 1104 and abdomen area 1106. This improves safety, and reduces pressure on the chest and abdomen area.

As a safety mechanism, when the upper body pressure is activated, the lower body pressure will deflate and vice versa. This will ensure the respiratory motion is not fully restricted. This means that even though breathing is somewhat restricted during an anxiety attack, there is still a safety margin for adequate breathing.

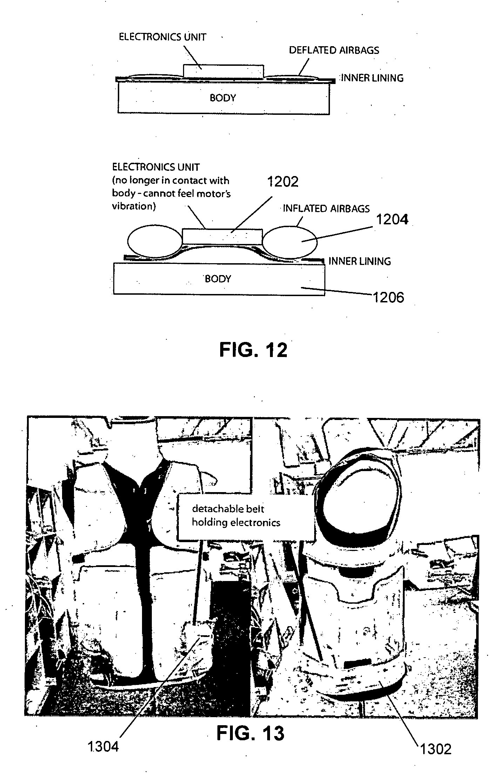

Figure 12 shows by careful positioning the pump 1202, when the airbags 1204 surrounding it are inflated, the vibration can be insulated from the user 1204. This may improve the user experience and may reduce noise level.

The master unit which comprises the controller is separated from the slave unit which comprises the air pump to minimise air chambers that will contribute to propagating the noise when there is a vibrational source. The slave unit is separated from the user by the bladders. Thus the air pump is as small and as isolated as possible to minimise the amount of noise produced. The slave pump may also have an o ring secured around the casing to reduce vibration and/or noise.

Figure 13 shows a "belt" 1302 that contains all the electronics 1304. It is designed to be easily removed from the jacket. The belt 1302 will be attached to the jacket by fasteners. The electronics 1304 are easily removed for washing and maintenance.

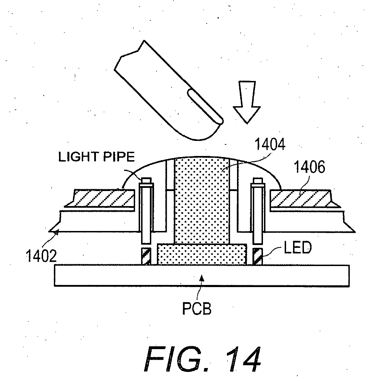

Figure 14 shows an overhanging shaped casing 1402 feature that holds the power button 1404 and the casing close to the fabric 1406. The button 1404 protrudes out of the fabric 1406 for easy locating and pressing. A button cover may also be provided to reduce accidental actuation of the power button 1404 by children who are self - stimulating (eg. rocking forward and backwards, jumping and falling flat on the ground). An anti flex tube may also be slotted into the plunger to cushion against accidental flexing of the casing that actuates the power button 1404.

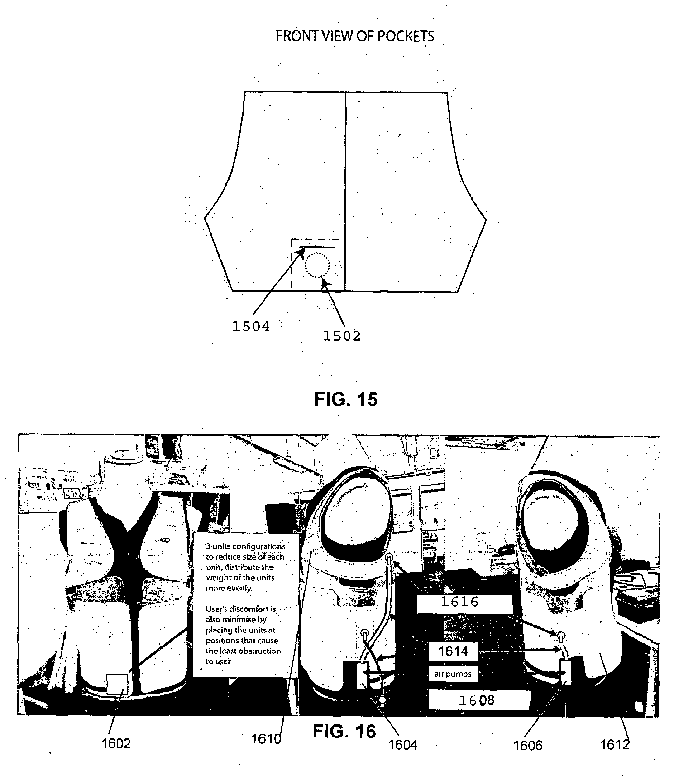

Figure 15 shows the LED lights indicators 1502 concealed within a small pocket 1504. The LED lighting 1502 is less obvious to people other than the jacket user or someone who is in close proximity.

Figure 16 shows the electronic components split into 3 locations: the main controller unit 1602 is placed near the main zip and each pump unit 1604, 1606 is placed near the waist of the user on opposite sides. This distribution minimises discomfort to the user while they are doing their daily activities. It reduces bulges on the soft outer fabric by placing them in non-obvious positions. It better distributes the weight of the system around the user's body to improve usability.

Figure 16 also shows the connection between the pump unit 1604 and the upper body bladder 1610 and the pump unit 1606 and the lower body bladder 1612. Air tubes 1614 are provided from the pump units 1604, 1606 to each of the bladder connectors 1616. The pressure sensor should be positioned as close as possible to the air pump units 1604, 1606. This is to minimize the length of the tube used for space considerations and this will impact on the size of the circuit box. Size of circuit box affects the user experience when wearing the jacket. The bladder connectors 1616 should be positioned at the sides of the body, to make use of the curvature of the waist to hide the protrusion of the pump inlet. A mechanical release valve 1608 may also be provided for the lower body bladder 1612.

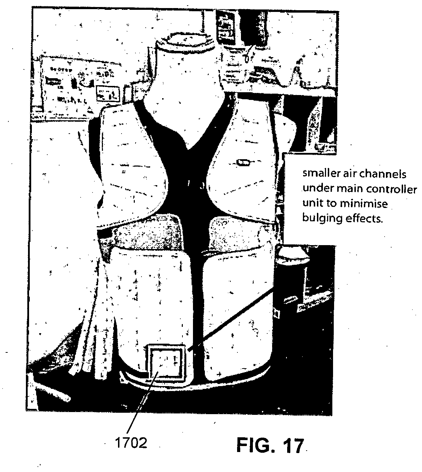

Figure 17 shows the airbag channels 1702 under the main controller unit are smaller so as to reduce the bulging effect caused by both the airbag inflation and the thickness of the main controller unit. This reduces bulging and makes the jacket more aesthetically pleasing.

All edges of the casings holding the electronics are designed to be more rounded so that there are no sharp edges which might make the users more uncomfortable especially when they are wearing them. A more rounded design also reduces the bulging look when the casings are lying against a soft fabric material.

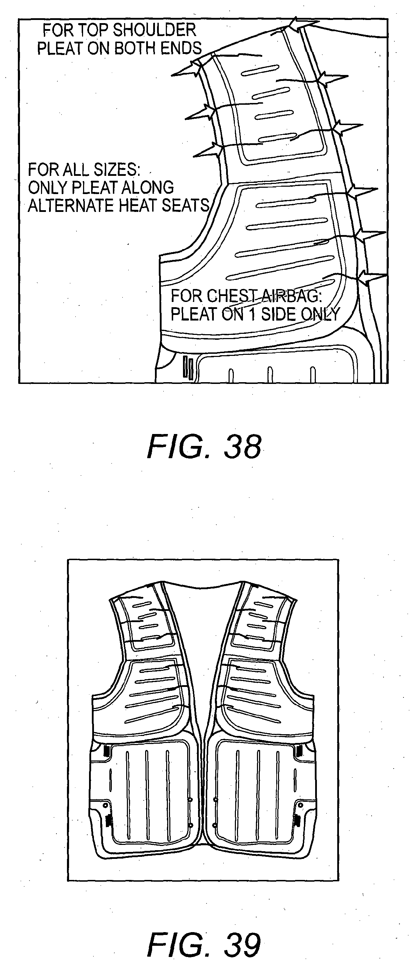

Figures 38 and 39 show the use pleating during sewing of the airbag to the inner fabric layer so as to minimise the "bulging" effect of the jacket. The pleating allows the airbag to be sewn in a "compressed" state, and thus will reduce the amount of pulling by the airbag on the fabric as it inflates. The shoulder airbag may have pleats on either edge as shown in Figure 38. The chest airbag may only have pleats on the outer edge as shown in Figure 39.

### Stimmina situations at home or in the outdoors

When at home or outdoors, the child can get hyperactive or start stimming. When this happens, the parent can use the remote app to apply deep pressure therapy from the jacket to calm down the child (which the child has already being wearing as the jacket is designed to be an everyday wear or would be instructed to wear the jacket by the parent). However, there will be stimming situations whereby the child is not wearing the jacket or the child would not want to put on the jacket.

### Pre empting stimming situations

• *Habitual*

Some stimming situations may be pre-empted by enticing the child to put on the jacket habitually as an everyday wear and using the pressure therapy regularly as part of their routine.

• *Self recognition*

The child also can be trained to recognise that when they are getting anxious, they will ask for or put on the jacket.

• *Self regulation*

For higher functioning children, if they recognise that they are getting anxious or they just like the pressure therapy, they might control the app themselves and activate the pressure.

### Prevention of habituation to the deep pressure effect

If static pressure is applied during each session there may be a risk in some cases that the user can habitualise the deep pressure effect after a certain period of time. The smart phone controller app allows automatically varying pressure within a time period, thus reducing the habituation effect and maximising the long term benefits of the deep pressure.

### Detection of hyperactivity or stress level

Assuming that they are already wearing the jacket, when sensors on the jacket detect hyperactivity or high stress level of the child, a notification will be sent to the parent or therapist. They will then have the option to start the pressure therapy or use other methods to calm down the child. An automatic triggering of the pressure therapy can also be activated when the hyperactivity or high stress level of the child is detected. This detection of hyperactivity or stress level offers convenience to the parents as they might not be always aware that their children are getting hyperactive or anxious. Early detection also allows the caregivers to intervene before a full attack occurs.

### Logging and sharing of pressure profiles used

The pressure profile used for the child is logged and can be anonymously shared with other users so that other parents and therapists will know what kind of pressure profile might be effective for a child with a particular profile.

Logging and sharing of care givers comments

The comments entered by caregivers before, during and after the pressure therapy sessions can be logged and be anonymously shared with other users so that other parents and therapists will understand better the effects of a certain pressure therapy on a child with a particular profile.

Therapist / teacher controlled

When the therapist or teacher is controlling the jacket, a parent will still be able to remotely supervise the pressure therapy sessions.

Teacher / therapist - multiple kids

As teachers and therapists sometimes conduct one teacher or therapist to many children (3 to 5 children) classes or therapy sessions, there are situations whereby multiple children can get hyperactive or start slimming at the same time. When this happens, the teacher or therapist can use their app to remotely apply deep pressure therapy to these children all at once to calm them down simultaneously.

Software modules

The system includes custom designed software in the app, on the cloud server and the remote app for the therapist. Each of the software modules will now be described in more detail.

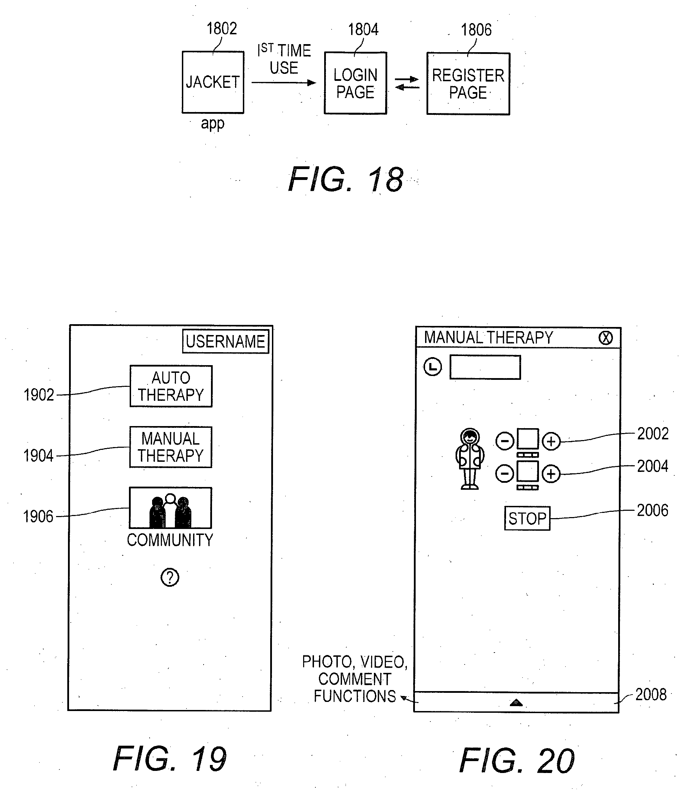

Figures 18 to 30 show screen shots of the app user interface.

1. As shown in Figure 18, when the user opens the App 1802 on a smartphone for the first time, it will prompt the user to either login 1804 (if user has already registered) or register 1806 a new user. Subsequently, by opening the App, as shown in Figure 19 user is brought to the main menu. From the main menu, users can perform the following functions (not limited to):

    a. Auto therapy 1902
    b. Manual therapy 1904
    c. Accessing the community 1906

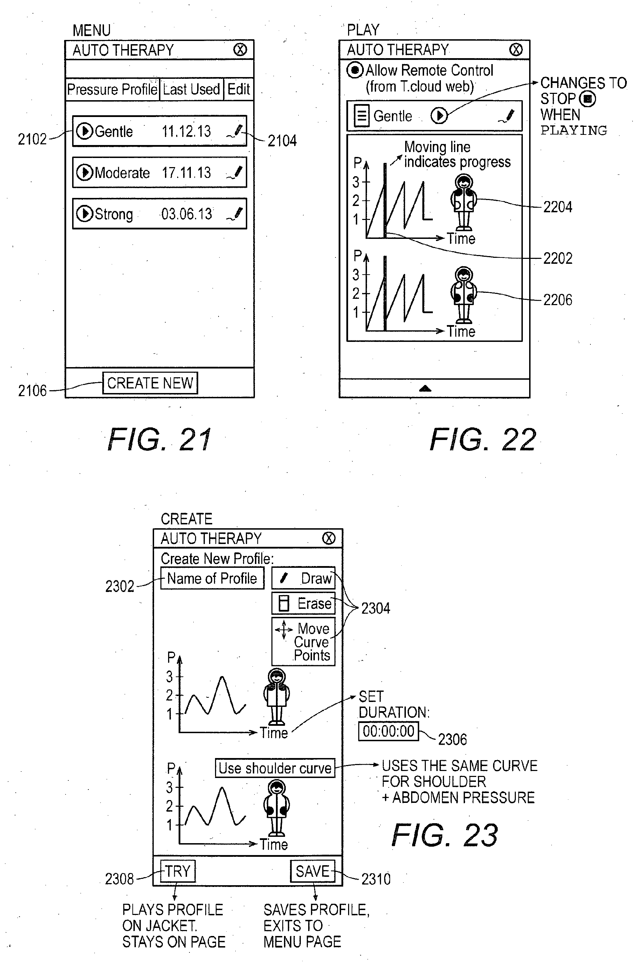

2. Manual therapy is shown in Figure 20 and relates to the control of the pressure levels and pressure areas of the jacket. The control allows the user to select the specific area (for instance shoulders 2002 and/or abdomen 2004) and the specific pressure levels to be applied to that area (for instance soft, medium or hard). The users can also stop 2006 the therapy at any time. From this view, at any time, the users can also take a picture, video or give comments by dragging up the bottom 2008 of the window.
3. Auto therapy, shown in Figure 21 relates to the playback of pre-set pressure profiles. Pressure profiles consist of the duration of the therapy and the variation of the pressure levels relative to time for each available pressure areas. In one instance, selecting the 'Gentle' pressure profile 2102 brings the user to the detailed view of the therapy shown in Figure 22.
4. Upon starting the therapy in Figure 22, user sees a moving vertical line 2202 which indicates progress along the 'Pressure versus time' graphs. The two graphs correspond to the two different areas 2204,2206 on the body where pressure can be applied. User can also edit 2104 the details in a pressure profile or create a new 2106 pressure profile.
5. As shown in Figure 23 when create or edit pressure profile is selected, user can change the following details:

    a. Name of profile 2302
    b. Pressure versus time graph: a selection of options 2304 are available to help user create or edit this graph, for example, users can draw the curves using their finger via the touchscreen
    c. Duration of the therapy 2306
    After these details are set, users have the option of previewing or trying it 2308, and saving it 2310 for future use.

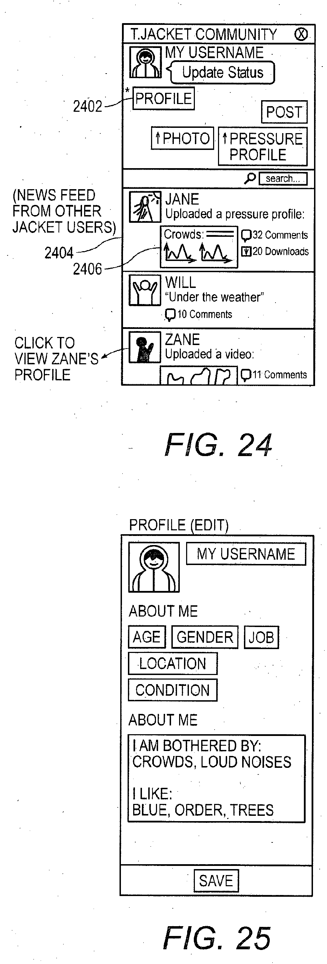

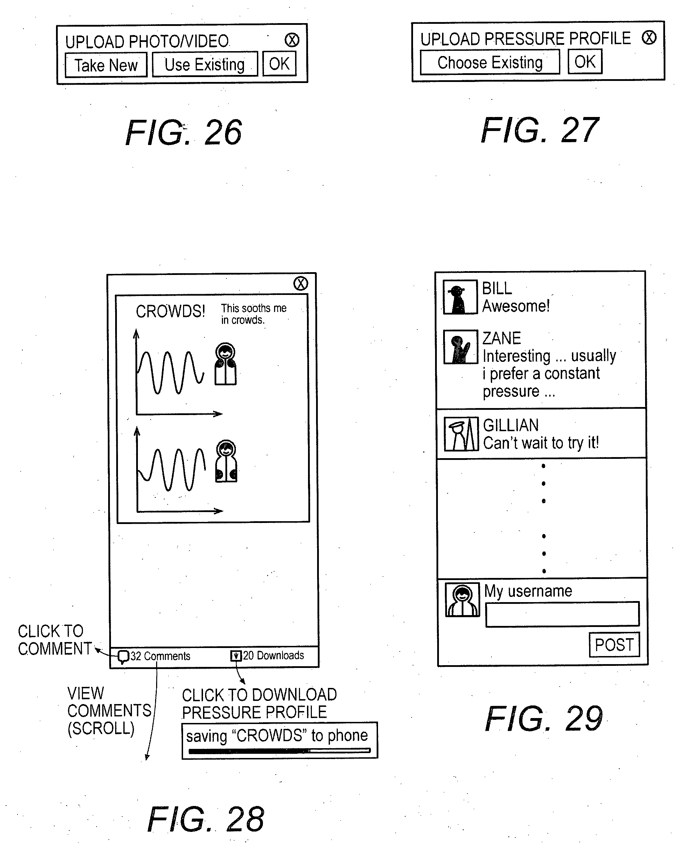

6. From the main page, user can access the Users Community 1906. Figure 24 shows by selecting the Profile button 2402, user can set the user profile as shown in Figure 25. These contain information related, but not limited, to the users that is relevant in understanding how the use of the jacket might benefit him.
7. Through this Community, user can interact with other users, share their status and other information such as pictures, uploading photos or videos as shown in Figure 26 and uploading pressure profiles as shown in Figure 27.

User can also view and access the information shared by other users via the Newsfeed column 2404. In one instance, user can view the pressure profile 2406 of another user and download the pressure profile for his or her own use as shown in Figure 28.

8. Users in the community can provide comments in relation to a pressure profile as shown in Figure 29 and see other users' comments on any of the items that are shared.

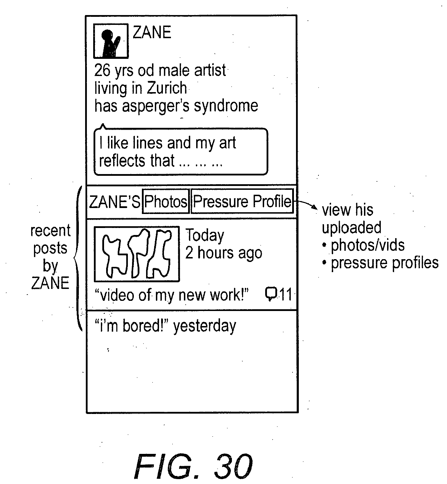

9. Users in the community can view others users' profile and the posts, photos, videos and pressure profiles that have been previously shared as shown in Figure 30.

Cloud Architecture

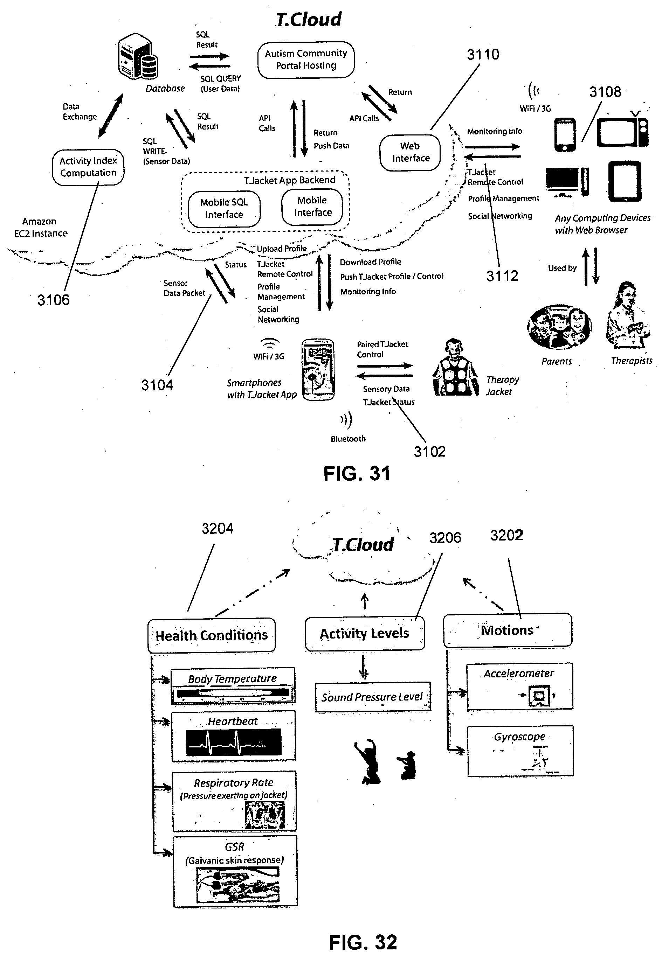

Figure 31 shows the data flow between the various software modules and the cloud Server. The child's motions are sensed 3102 and then uploaded 3104 to the cloud via a Bluetooth-paired smartphone's 3G or Wi-Fi connection. The motion data will be batch processed into an indicative activity index 3106 on the cloud, thus giving the therapist an idea of the child's activity level. Through a tablet or smartphone 3108 that has connectivity to the Internet, therapist can view the activity level in a graphical form 3110 and then make decision to set and activate deep pressure touch 3112 for the child. The changes made will be sent to the cloud and pushed to the smartphone that is controlling the corresponding garment.

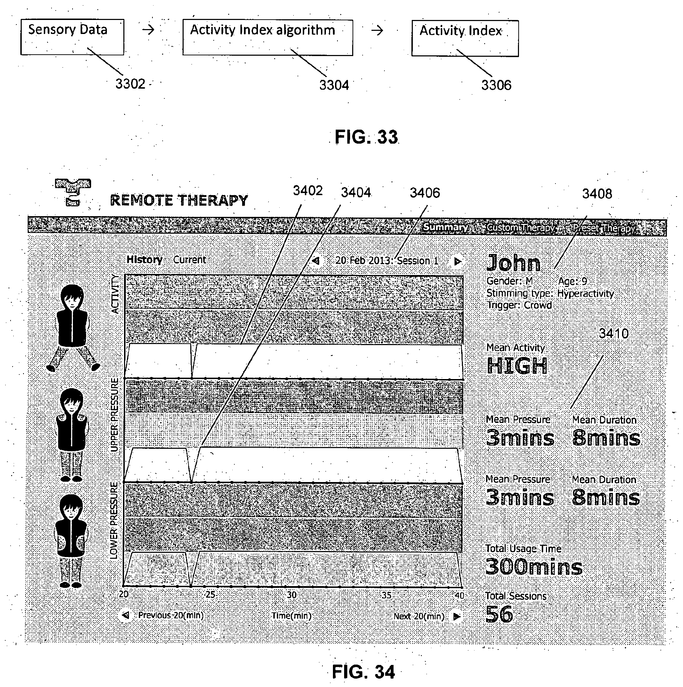

Figure 32 shows the types of data that can be continuously recorded (for instance motion 3202, health 3204 and activity 3206) from the user. This data 3302 can then be used by the algorithm 3304 as shown in Figure 33 to calculate the activity index 3306. An example of the algorithm 3304 is defined as below:

$$Activity\ Index = \omega_1(\Delta T) + \omega_2(\Delta R) + \omega_3(\Delta SPL) + \omega_4(D) + c$$

$\omega_n$ : Weight for each Parameter
$\Delta T$ : Changes in Body Temperature
$\Delta R$ : Changes in Heartbeat Rate
$\Delta SPL$ : Changes in Sound Pressure Level
$D$ : Displacement
$c$ : Calbration Constant

An activity index may be useful for therapists and parents as it can relate to user's arousal level and gives indication about condition of the children. Through monitoring Activity Index variations pattern, therapists and parents can choose to activate pressure profiles or soothe the user in person.

Autism Community Portal

Current data collection for most home and school based autism programs consists of paper and pencil with team members exchanging notes on the children and effective techniques. This makes it difficult to perform meaningful data analysis.

Examples of the present invention, not being part of the invention, may provide an easy, digital system to automatically record and transmit important data for children wearing the jacket. This may allow teachers, therapists and parents to have access to appropriate data anytime and anywhere, thus enabling everyone to work towards the same goals using the same kind of treatment. The Autism Community Portal may enable sharing of information about different children with autism, their profiles and treatments received. This may promote a community driven approach towards the best practice among individual families and caregivers.

The Autism Community Portal may be access using a Web User Interface (Web UI) or a Smart Phone app (app UI). When a teacher, therapist or parent logs in, they are presented with a list of user's they are approved to view and/or control. For each user the web UI presents three different window views for users.

Figure 34 shows the summary page. On the graphs at the centre Historical activity 3402 and pressure levels and location applied 3404 are summarised. Data for different sessions can be selected based on date and session number 3406, and the graph is regenerated. The selected user is shown in top right hand corner together with profile data 3408. The average pressure and duration of pressure application is also displayed 3410.

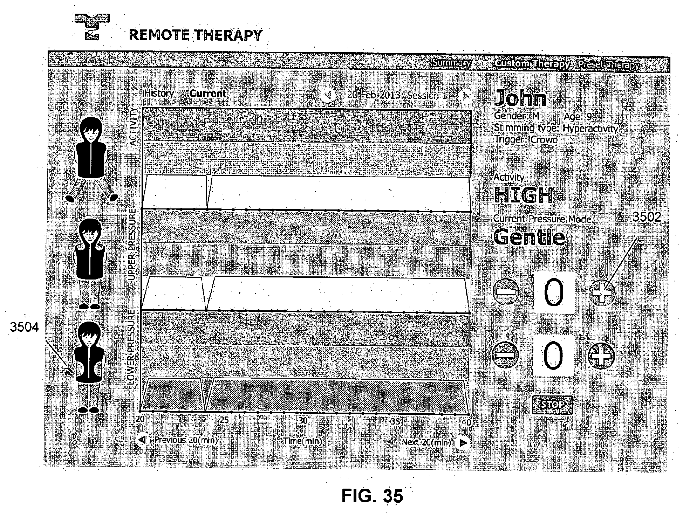

For custom therapy, shown in Figure 35, the right hand column of the window changes to reveal the pressure control interface. Users can select the specific pressure levels 3502 at specific pressure areas 3504 to be applied. Users can stop the therapy at any time.

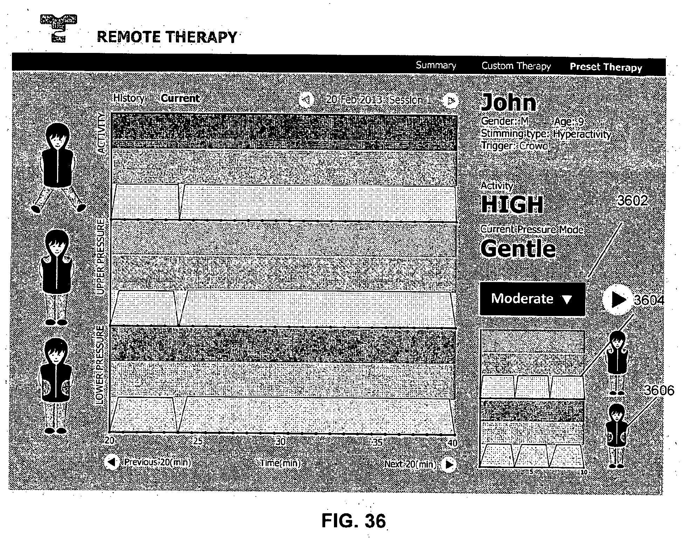

For Preset Therapy, shown in Figure 36, users can select from a range of preset therapy or pressure profiles 3602. In this view, the right hand column of the window reveals the pressure application 3604 varying with time that will be applied to the different areas 3606 on the body of the user.

Whilst exemplary embodiments of the invention have been described in detail, many variations are possible within the scope of the invention as will be clear to a skilled reader. For example the garment could be adapted for use as a mobile and wearable massage system, eg: a jacket, that can be used by the individuals anytime, anywhere. Such a system will provide a mobile, convenient and wearable touch stimulation to calm and provide a soothing effect for individuals on the go.

Possible usage scenarios include using it when they are tired or in danger of Deep Vein Thrombosis due to their lack of movement, for example in the office, travelling or on long flights. Another scenario includes the elderly using it as it might be easier to provide massage through a wearable compared to conventional static massage products due to their immobility. Remote therapy by professional therapists can also be applied on elderly at homes.

Massage hardware requirements

1. more targeted pressure points, especially on the upper body like the shoulders, neck and upper arms, and also on the lower back region. As of now, the air bladders are designed to give a wrap around pressure over a larger surface area on the user's body. However, for the massage application, common massage pressure areas will be targeted instead of an all round pressure. This may require more independently controlled bladders in different locations.

2. By varying the airflow speed, airflow channels configurations, pressure duration, and pressure intensity, various massaging effects like kneading, pushing, stroking can be simulated.

Massage software requirements

1. Preset pressure patterns for massage instead of autism
2. Crowd sharing massage profiles (instead of deep pressure therapy profiles) shared over the cloud by various users.
3. Community discussion on the effectiveness of the massage for various ailments (eg. pains in joints, arthritis)
4. Remote massage therapy done by therapists on home patients (eg. elderly) as an after hospitalization therapy.
5. Tracking physiological activity like heart rate, stress, movement to detect when the user should be given a massage. Auto activation of the massage can also be done.

**Claims**

1. A garment (200) for treating a sensory disorder, the garment (200) comprising:

   at least two zones,
   at least two air bladders (202), at least one air bladder (202) located within each zone, the air bladders (202) configured to constrict the torso of a user,
   a first sensor (210) configured to detect the pressure in the air bladders,
   a second sensor (210) configured to track at least one of activity, motion, and physiological parameters of the user, and
   a controller (208) configured to communicate with a mobile device application (300) to allow the user to control the pressure in each air bladder (202) independently from one another according to predetermined criteria,
   wherein the controller (208) comprises at least a master processor (302) and a master pump (204) for a first of the at least two air bladders (202), and a slave processor (406) and a slave pump (402) for each remaining air bladder (202), and
   wherein the controller (208) is configured to vary the pressure in each air bladder (202) based on an activity index (205a) calculated using a weighted sum of one or more of the following: changes in body temperature, changes in heartbeat rate, changes in sound pressure level, and displacement of the user.

2. The garment in claim 1 wherein the garment is configured for massaging the user to calm the user.

3. The garment in any preceding claim wherein controlling the pressure in each air bladder independently according to the predetermined criteria includes automatic variation of the independent pressures in the air bladders within a time period.

4. The garment in any preceding claim wherein the air bladders are configured to minimise the garment lifting up when inflated by maximising the constriction around the torso and anchoring under the arm pit.

5. The garment in any preceding claim wherein the first sensor comprises a plurality of pressure transducers, each transducer providing a signal to the controller indicative of the pressure within a specific one of the at least two air bladders.

6. The garment in any preceding claim wherein the controller comprises at least a master processor, one or more slave processors, one or more pumps and/or valves, and tubing, wherein the connection of the tubing between the pumps and the air bladders is customizable by the user.

7. The garment in any preceding claim further comprising a plurality of actuators for producing an air pressure in each of the air bladders depending on power supplied by the controller.

8. The garment in any preceding claim wherein the controller is attached to a removable belt, and wherein the controller is distributed in separate units along the removable belt.

9. The garment in any preceding claim further comprising a power button configured to be easily located and pressed and with light indicators that are obscured to third parties other than the user.

10. The garment in claim 9 further comprising a button cover configured to reduce accidental actuation of the power button by a user during an attack or stimming.

11. The garment in any preceding claim wherein each air bladder comprises a configuration of air channels for reducing bulging and/or constricting the torso, said configuration being dependent on the position of the air bladder with respect to the torso.

12. The garment in any preceding claim wherein the air bladders comprise pleating along one or more edges configured to reduce bulging.

13. A mobile device application comprising code means adapted to control a garment according to any of claims 1 to 12, when said application is run on a mobile device.

**Patentansprüche**

1. Kleidungsstück (200) zum Behandeln einer Sinnesstörung, wobei das Kleidungsstück (200) Folgendes umfasst:

wenigstens zwei Zonen,
wenigstens zwei Luftblasen (202), wenigstens eine innerhalb jeder Zone angeordnete Luftblase (202), wobei die Luftblasen (202) dafür konfiguriert sind, den Rumpf eines Benutzers einzuengen,
einen ersten Sensor (210), der dafür konfiguriert ist, den Druck in den Luftblasen zu erfassen,
einen zweiten Sensor (210), der dafür konfiguriert ist, wenigstens eines von Aktivität, Bewegung und physiologischen Parametern des Benutzers zu verfolgen, und
ein Steuergerät (208), das dafür konfiguriert ist, mit einer Mobilgerät-Anwendung (300) zu kommunizieren, um es dem Benutzer zu ermöglichen, den Druck in jeder Luftblase (202) unabhängig voneinander entsprechend vorbestimmten Kriterien zu steuern,
wobei das Steuergerät (208) wenigstens einen Hauptprozessor (302) und eine Hauptpumpe (204) für eine erste der wenigstens zwei Luftblasen (202) und einen Nebenprozessor (406) und eine Nebenpumpe (402) für jede verbleibende Luftblase (202) umfasst und
wobei das Steuergerät (208) dafür konfiguriert ist, den Druck in jeder Luftblase (202) auf Grundlage eines Aktivitätsindex (205a) zu steuern, der unter Verwendung einer gewichteten Summe eines oder mehrerer von Folgendem berechnet wird: Änderungen bei der Körpertemperatur, Änderungen bei der Herzschlagfrequenz, Änderungen beim Schalldruckpegel und Verlagerung des Benutzers.

**2.** Kleidungsstück nach Anspruch 1, wobei das Kleidungsstück zum Massieren des Benutzers konfiguriert ist, um den Benutzer zu beruhigen.

**3.** Kleidungsstück nach einem der vorhergehenden Ansprüche, wobei das unabhängige Steuern des Drucks in jeder Luftblase entsprechend den vorbestimmten Kriterien eine automatische Veränderung der unabhängigen Drücke in den Luftblasen innerhalb eines Zeitraums einschließt.

**4.** Kleidungsstück nach einem der vorhergehenden Ansprüche, wobei die Luftblasen dafür konfiguriert sind, das Anheben des Kleidungsstücks, wenn sie aufgeblasen werden, durch Maximieren der Einengung um den Rumpf und Verankern unter der Achselhöhle zu minimieren.

**5.** Kleidungsstück nach einem der vorhergehenden Ansprüche, wobei der erste Sensor eine Vielzahl von Druckwandlern umfasst, wobei jeder Wandler ein Signal für das Steuergerät bereitstellt, das den Druck innerhalb einer spezifischen der wenigstens zwei Luftblasen anzeigt.

**6.** Kleidungsstück nach einem der vorhergehenden Ansprüche, wobei das Steuergerät wenigstens einen Hauptprozessor, einen oder mehrere Nebenprozessoren, eine oder mehrere Pumpen und/oder Ventile und Verrohrung umfasst, wobei die Verbindung der Verrohrung zwischen den Pumpen und den Luftblasen durch den Benutzer anpassbar ist.

**7.** Kleidungsstück nach einem der vorhergehenden Ansprüche, das ferner eine Vielzahl von Stellantrieben zum Erzeugen eines Luftdrucks in jeder der Luftblasen in Abhängigkeit von einer durch das Steuergerät zugeführten Leistung umfasst.

**8.** Kleidungsstück nach einem der vorhergehenden Ansprüche, wobei das Steuergerät an einem abnehmbaren Gürtel befestigt ist und wobei das Steuergerät in gesonderten Einheiten entlang des abnehmbaren Gürtels verteilt ist.

**9.** Kleidungsstück nach einem der vorhergehenden Ansprüche, das ferner einen Ein-/Aus-Knopf umfasst, der dafür konfiguriert ist, leicht aufzufinden und zu drücken zu sein, und mit Lichtanzeigen, die für von dem Benutzer verschiedene Dritte verborgen sind.

**10.** Kleidungsstück nach Anspruch 9, das ferner eine Knopfabdeckung umfasst, die dafür konfiguriert ist, eine versehentliche Betätigung des Ein-/Aus-Knopfs durch einen Benutzer während eines Anfalls oder des Stimmings (selbststimulierenden Verhaltens).

**11.** Kleidungsstück nach einem der vorhergehenden Ansprüche, wobei jede Luftblase eine Konfiguration von Luftkanälen zum Verringern von Vorwölben und/oder Einengen des Rumpfes umfasst, wobei die Konfiguration abhängig ist von der Position der Luftblase in Bezug auf den Rumpf.

**12.** Kleidungsstück nach einem der vorhergehenden Ansprüche, wobei die Luftblasen eine Faltung entlang einer oder mehrerer Kanten umfassen, die dafür konfiguriert ist, ein Vorwölben zu verringern.

**13.** Mobilgerät-Anwendung, die Codemittel umfasst, die dafür eingerichtet sind, ein Kleidungsstück nach einem der Ansprüche 1 bis 12 zu steuern, wobei die Anwendung auf einem Mobilgerät laufen gelassen wird.

**Revendications**

**1.** Vêtement (200) pour traiter un trouble sensoriel, le vêtement (200) comprenant :

au moins deux zones,
au moins deux vessies d'air (202), au moins une vessie d'air (202) étant située dans chaque zone, les vessies d'air (202) étant configurées pour resserrer le torse d'un utilisateur,
un premier capteur (210) configuré pour détecter la pression dans les vessies d'air,
un second capteur (210) configuré pour suivre au moins un élément parmi une activité, un mouvement, et des paramètres physiologiques de l'utilisateur, et
un système de commande (208) configuré pour communiquer avec une application de dispositif mobile (300) afin de permettre à l'utilisateur de contrôler la pression dans chaque vessie d'air (202) indépendamment l'une de l'autre selon des critères prédéterminés,

dans lequel le système de commande (208) comprend au moins un processeur principal (302) et une pompe principale (204) pour une première desdites au moins deux vessies d'air (202), et un processeur auxiliaire (406) et une pompe auxiliaire (402) pour chaque vessie d'air restante (202), et

dans lequel le système de commande (208) est configuré pour varier la pression dans chaque vessie d'air (202) sur la base d'un indice d'activité (205a) calculé en utilisant une somme pondérée d'un ou plusieurs des éléments suivants : changements de température corporelle, changements du rythme cardiaque, changements du niveau de pression sonore, et déplacement de l'utilisateur.

2. Vêtement selon la revendication 1, dans lequel le vêtement est configuré pour masser l'utilisateur afin de le détendre.

3. Vêtement selon l'une quelconque des revendications précédentes, dans lequel le contrôle de la pression dans chaque vessie d'air indépendamment selon les critères prédéterminés inclut la variation automatique des pressions indépendantes dans les vessies d'air dans un laps de temps donné.

4. Vêtement selon l'une quelconque des revendications précédentes, dans lequel les vessies d'air sont configurées de façon à minimiser le soulèvement du vêtement lorsqu'elles sont gonflées, en optimisant la constriction autour du torse et en se fixant sous l'aisselle.

5. Vêtement selon l'une quelconque des revendications précédentes, dans lequel le premier capteur comprend une pluralité de transducteurs de pression, chaque transducteur fournissant un signal au système de commande indicatif de la pression dans une spécifique desdites au moins deux vessies d'air.

6. Vêtement selon l'une quelconque des revendications précédentes, dans lequel le système de commande comprend au moins un processeur principal, un ou plusieurs processeur(s) auxiliaire(s), une ou plusieurs pompe(s) et/ou soupape(s), et un tubage, dans lequel le raccordement du tubage entre les pompes et les vessies d'air est personnalisable par l'utilisateur.

7. Vêtement selon l'une quelconque des revendications précédentes, comprenant en outre une pluralité d'actionneurs pour produire une pression d'air dans chacune des vessies d'air en fonction de l'alimentation fournie par le système de commande.

8. Vêtement selon l'une quelconque des revendications précédentes, dans lequel le système de commande est fixé à une courroie amovible, et dans lequel le système de commande est distribué en unités séparées le long de la courroie amovible.

9. Vêtement selon l'une quelconque des revendications précédentes, comprenant en outre un bouton d'alimentation configuré pour être facilement localisé et enfoncé et avec des indicateurs lumineux qui sont occultés aux tiers autres que l'utilisateur.

10. Vêtement selon la revendication 9, comprenant en outre un capot de bouton configuré pour réduire l'actionnement accidentel du bouton d'alimentation par un utilisateur pendant une attaque ou une auto stimulation.

11. Vêtement selon l'une quelconque des revendications précédentes, dans lequel chaque vessie d'air comprend une configuration de canaux d'air pour réduire le gonflement et/ou resserrer le torse, ladite configuration dépendant de la position de la vessie d'air par rapport au torse.

12. Vêtement selon l'une quelconque des revendications précédentes, dans lequel les vessies d'air comprennent le plissage le long d'un ou plusieurs bord(s) configuré(s) pour réduire le gonflement.

13. Application de dispositif mobile comprenant un moyen de code adapté pour contrôler un vêtement selon l'une quelconque des revendications 1 à 12, quand ladite application est exécutée sur un dispositif mobile.

**FIG. 1**

JACKET INTERACTION DIAGRAM

FIG. 2a

JACKET INTERACTION DIAGRAM

USER       APP       CLOUD       JACKET       APP (REMOTE)       THERAPIST

Check Connection

204

Connection Status

Update Status    (3G/Wifi)

Select Remote
Control Function    205

Select/Upload    Therapy Profile

Push Profile

(3G/Wifi)

Status

Control (BT)

Status
Req. Motion Data

Motion Data

Log Motion Data    (3G/Wifi)

Activity Index
Computation

View Activity    205a

Req. Motion Data/Activity Index

Motion Data/Activity Index    Graph Display

Change Profile    205b

Upload Profile Changes

Push Profile

(3G/Wifi)

Status

Control (BT)

Status
Req. Motion Data

Motion Data

Log Motion Data    (3G/Wifi)

Activity Index
Computation

View Activity    205c

Req. Motion Data/Activity Index

Motion Data/Activity Index    Graph Display

Therapy End

*FIG. 2b*

FIG. 3

FIG. 4

```
                    ┌─────────────┐
                    │    Start    │
                    └─────────────┘                          500
                           │                                  /
                           ▼                                 ↙
        ┌──────────────────────────────────────┐ ─┐
        │ Initialize General Purpose Input/Output│  │
        └──────────────────────────────────────┘  │
                           │                       │
                           ▼                       │
        ┌──────────────────────────────────────┐  │
        │        Initialize Interrupt Timer      │  ├── 502
        └──────────────────────────────────────┘  │
                           │                       │
                           ▼                       │
        ┌──────────────────────────────────────┐  │
        │        Initialize Air Bladder State    │  │
        │          Target Pressure = 0           │  │
        └──────────────────────────────────────┘  │
                           │                       │
                           ▼                       │
        ┌──────────────────────────────────────┐  │
        │           Power-on Sequence            │  │
        └──────────────────────────────────────┘  │
                           │                       │
                           ▼                       │
        ┌──────────────────────────────────────┐  │
        │             Initialize I2C             │  │
        │             Initialize IMU             │  │
        │             Initialize UART            │  │
        └──────────────────────────────────────┘ ─┘
                           │
                           ▼
        ┌──────────────────────────────────────┐
        │   Schedule Air Bladder Update Routine  │
        │   Schedule Air Bladder Check Routine   │── 504
        │       Schedule IMU Read Routine        │
        │  Schedule UART Communication Routine   │
        │         Schedule Other Routine         │
        └──────────────────────────────────────┘     506
                           │                          /
                           ▼
        ┌──────────────────────────────────────┐◄─┐
        │             Check Schedule             │  │  508
        └──────────────────────────────────────┘  │  /
                           │                       │
                           ▼                       │
        ┌──────────────────────────────────────┐◄─┘
        │             Perform Routine            │
        └──────────────────────────────────────┘
```

**FIG. 5**

Air Bladder Update Routine
Routine performed every 100ms or less

**FIG. 6**

CP: Current Pressure
TP: Target Pressure
T1: Overshoot Threshold
T2: Overpressure Threshold
T3: Undershoot Threshold

FIG. 7

**FIG. 8**

802

804

902

904

906

**FIG. 9**

**FIG. 10**

air bag applies
inwards pressure
towards body

**FIG. 11**

ELECTRONICS UNIT

DEFLATED AIRBAGS

INNER LINING

BODY

ELECTRONICS UNIT
(no longer in contact with
body - cannot feel motor's
vibration)

1202

INFLATED AIRBAGS

1204

INNER LINING

BODY

1206

## FIG. 12

detachable belt
holding electronics

## FIG. 13

1304

1302

LIGHT PIPE

1404

1406

1402

LED

PCB

FIG. 14

FRONT VIEW OF POCKETS

1504    1502

**FIG. 15**

3 units configurations to reduce size of each unit, distribute the weight of the units more evenly.

User's discomfort is also minimise by placing the units at positions that cause the least obstruction to user

1616

1614

air pumps

1608

1602    1610    **FIG. 16**    1604    1606    1612

smaller air channels
under main controller
unit to minimise
bulging effects.

1702      **FIG. 17**

1802　　IST TIME　　1804　　　1806
　　　　　USE
JACKET　→　LOGIN　⇄　REGISTER
　　　　　　　PAGE　　　PAGE
app

## FIG. 18

USERNAME

1902 — AUTO THERAPY

1904 — MANUAL THERAPY

1906 — COMMUNITY

?

## FIG. 19

MANUAL THERAPY　⊗

Ⓛ ▢

⊖ ▢ ⊕ — 2002
⊖ ▢ ⊕ — 2004

STOP — 2006

PHOTO, VIDEO, COMMENT FUNCTIONS ← ▲ — 2008

## FIG. 20

## FIG. 21

**MENU**

AUTO THERAPY ⊗

| Pressure Profile | Last Used | Edit |
|---|---|---|

2102 — ▶Gentle   11.12.13   ✎ — 2104

▶Moderate   17.11.13   ✎

▶Strong   03.06.13   ✎

2106 — CREATE NEW

## FIG. 22

**PLAY**

AUTO THERAPY ⊗

◉ Allow Remote Control
(from T.cloud web) → CHANGES TO STOP ◉ WHEN PLAYING

☰ Gentle  ▶  ✎

Moving line indicates progress

P
3
2 — 2204
1
→ Time — 2202

P
3
2 — 2206
1
→ Time

## FIG. 23

**CREATE**

AUTO THERAPY ⊗

Create New Profile:

2302 — Name of Profile

✎ Draw
⊟ Erase — 2304
⬍ Move Curve Points

P
3
2
1
→ Time

→ SET DURATION:
00:00:00 — 2306

P
3
2
1
→ Time

Use shoulder curve → USES THE SAME CURVE FOR SHOULDER + ABDOMEN PRESSURE

2308 — TRY   SAVE — 2310

PLAYS PROFILE ON JACKET. STAYS ON PAGE

SAVES PROFILE, EXITS TO MENU PAGE

T.JACKET COMMUNITY ⊗

MY USERNAME
Update Status

2402 — *PROFILE

POST

↑PHOTO | ↑PRESSURE PROFILE

🔍 search...

(NEWS FEED FROM OTHER JACKET USERS)

2404 —

2406 —

JANE
Uploaded a pressure profile:

Crowds: 💬32 Comments
📥20 Downloads

WILL
"Under the weather"
💬10 Comments

CLICK TO VIEW ZANE'S PROFILE

ZANE
Uploaded a video:
💬11 Comments

## FIG. 24

PROFILE (EDIT)

MY USERNAME

ABOUT ME

AGE | GENDER | JOB

LOCATION

CONDITION

ABOUT ME

I AM BOTHERED BY:
CROWDS, LOUD NOISES

I LIKE:
BLUE, ORDER, TREES

SAVE

## FIG. 25

UPLOAD PHOTO/VIDEO    ⊗
| Take New | Use Existing | OK |

## FIG. 26

UPLOAD PRESSURE PROFILE  ⊗
| Choose Existing | OK |

## FIG. 27

⊗

CROWDS!    This sooths me in crowds.

CLICK TO
COMMENT

Q 32 Comments    ⬇ 20 Downloads

VIEW
COMMENTS
(SCROLL)

CLICK TO DOWNLOAD
PRESSURE PROFILE
| saving "CROWDS" to phone |

## FIG. 28

BILL
Awesome!

ZANE
Interesting ... usually
i prefer a constant
pressure ...

GILLIAN
Can't wait to try it!

My username
[                    ]
| POST |

## FIG. 29

ZANE

26 yrs od male artist
living in Zurich
has asperger's syndrome

I like lines and my art
reflects that ... ... ...

recent
posts
by
ZANE

ZANE'S Photos Pressure Profile

Today
2 hours ago

"video of my new work!"  ⬭11

"i'm bored!" yesterday

view his
uploaded
• photos/vids
• pressure profiles

*FIG. 30*

## T.Cloud

**FIG. 31**

**FIG. 32**

Sensory Data → Activity Index algorithm → Activity Index

3302

3304

3306

**FIG. 33**

REMOTE THERAPY     3402  3404    3406                    3408

Summary   Custom Therapy   Preset Therapy

History  Current          ◄  20 Feb 2013 Session 1  ►    **John**

Gender: M      Age: 9
Stimming type: Hyperactivity
Trigger: Crowd

3410

Mean Activity

**HIGH**

Mean Pressure    Mean Duration

**3mins    8mins**

Mean Pressure    Mean Duration

**3mins    8mins**

Total Usage Time

**300mins**

20         25         30         35         40    Total Sessions

◄ Previous 20(min)        Time(min)        Next 20(min) ►   **56**

**FIG. 34**

FIG. 35

**FIG. 36**

*FIG. 37*

FOR TOP SHOULDER
PLEAT ON BOTH ENDS

FOR ALL SIZES:
ONLY PLEAT ALONG
ALTERNATE HEAT SEATS

FOR CHEST AIRBAG:
PLEAT ON 1 SIDE ONLY

*FIG. 38*

*FIG. 39*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2355767 A
- US 20040054306 A
- WO 20080314 A
- US 5437610 A
- WO 2011084709 A
- US 20120284898 A
- WO 2009114822 A
- KR 20120102434
- KR 20120078760
- US 2002089304 A
- US 2006242746 A
- KR 20120034095

- US 6086551 A
- US 2003074711 A
- US 2010199405 A
- US 2003230474 A
- CN 201541756
- US 2008222771 A
- US 2008222769 A
- US 2010292619 A
- US 20060122544 A
- US 6752771 B
- US 5490820 A